Europäisches Patentamt

European Patent Office

Office européen des brevets

(19)

(11) Publication number: **0 060 611**
A2

# EUROPEAN PATENT APPLICATION

(12)

(21) Application number: 82300398.3

(51) Int. Cl.³: **A 61 K 7/06, A 61 K 7/08**

(22) Date of filing: 27.01.82

(30) Priority: 31.01.81 GB 8103020

(43) Date of publication of application: 22.09.82
Bulletin 82/38

(84) Designated Contracting States: **BE CH DE FR GB IT LI NL**

(71) Applicant: **BEECHAM GROUP PLC, Beecham House Great West Road, Brentford Middlesex (GB)**

(72) Inventor: **Elpe, Raju Kattunilath, 38 Craneswater, Harlington Middlesex, UB3 5HP (GB)**

(74) Representative: **Cresswell, Thomas Anthony et al, European Patent Attorney Beecham Pharmaceuticals Great Burgh Yew Tree Bottom Road, Epsom Surrey KT18 5XQ (GB)**

(54) Medicated hair conditioner.

(57) A medicated foaming conditioner comprises

| | |
|---|---|
| zinc pyridinethione | — 0.01 - 5.0 % w/w |
| cationic suspending agent(s) | — qs |
| foam building agent(s) | — 0.1 - 15 % w/w |
| cationic conditioning agent(s) | — 0.1 - 10 % w/w |

and is useful for treating or preventing dandruff.

EP 0 060 611 A2

0060611

MEDICATED CONDITIONER

The present invention relates to hair care products and, in particular, to a medicated conditioner containing zinc pyridinethione as anti-dandruff agent.

It is well known that shampooing with conventional anionic surfactants removes oils from the hair, leaving it clean but in poor condition. This situation is exacerbated when medicated shampoos are used to control dandruff. To counteract these undesirable effects it is usual to use a cationic conditioning rinse which restores the gloss and manageability to the hair.

In European patent application 79301208.9 an improved hair conditioning agent containing zinc pyridinethione was described. This could be used in place of a conventional conditioner and with an unmedicated shampoo to give more effective dandruff control than a medicated shampoo.

When using shampoos, whether medicated or unmedicated, the consumer conventionally uses two separate applications of shampoo followed by one application of a conditioner. However, the second application of shampoo has little cleansing effect and may be unnecessary and wasteful, but is undertaken to achieve a firm lather to indicate that the hair is clean.

It has now been found that this waste may be avoided and a more effective cleaning, conditioning and dandruff control treatment may be applied using a medicated foaming conditioner.

Accordingly the present invention provides a medicated foaming conditioner composition for conditioning the hair and for clearing or preventing dandruff, which composition comprises:

zinc pyridinethione;
one or more cationic suspending agents;
one or more foam building agents; and, if necessary,
one or more cationic conditioning agents.

Conditioners according to the present invention may be used, instead of a second application of shampoo, to raise the firm lather which indicates the hair to be clean. This procedure leaves the hair in better and more manageable condition than two applications of shampoo and prevents wasting shampoo. It has the further advantage of requiring less time and effort than the use of two applications of shampoo followed by a conventional conditioner.

Conveniently, the conditioner comprises from 0.01 to 5.0% w/w zinc pyridinethione, preferably 0.1 to 1.0% w/w. The zinc pyridinethione is provided in conventional particulate form and is held in suspension by cationic suspending agents.

Suitable cationic suspending agents are natural or synthetic cationic gums, clays and gel-forming compounds including various gel-forming polymers, such as cellulose derivatives and certain proteins, which provide adequate viscosity and appropriate rheological properties to maintain the zinc pyridinethione in suspension. A particularly suitable suspending agent is Polymer JR 30M, a high molecular weight quaternised hydroxyethyl cellulose.

The foam-building agent may be a detergent, whether anionic, cationic, amphoteric or nonionic. Suitable cationic detergents include quaternary ammonium compounds such as cetyl trimethyl ammonium bromide or chloride. Suitable nonionic detergents include fatty acid ethanol amides. Suitable amphoteric detergents include N-alkyl betaines and N-lauryl myristyl-beta propionic acid. Suitable anionic detergents include sarcosinic acid detergents such as sodium lauroyl sarcosinate, which are preferred.

The conditioner suitably comprises 0.1 to 15% w/w of the foam building agent, preferably 1 to 5% w/w.

As a conditioner, the composition of the present invention should include one or more agents which are deposited on the hair shaft as a coating, or are substantive to the hair shaft and thereby impart the properties collectively or individually considered to indicate good hair condition, such as gloss, lustre, body and/or manageability. Conditioning agents are cationic surfactants and are well known in the hair-care art; suitable examples include quaternary ammonium compounds such as stearyl dimethyl benzyl ammonium chloride, distearyl dimethyl ammonium chloride, cetyl trimethyl ammonium chloride, cetyl ethyl morpholinium 2-etho-sulphate, mink-amido-propyl dimethyl 2-hydroxyethyl ammonium chloride and N-stearyl colamino formyl methyl pyridinium chloride or the corresponding bromides.

Preferred conditioning agents are stearyl dimethyl benzyl ammonium chloride and cetyl trimethyl ammonium chloride, and cetyl trimethyl ammonium bromide.

The conditioner suitably comprises 0.1% to 10% w/w of the cationic conditioning agent, preferably 1% to 5% w/w.

It will be appreciated that certain cationic polymers may perform the functions of both suspending agent and conditioning agent. When such a polymer is used as suspending agent in the compositions of the present invention it is not necessary to include a separate conditioning agent, although this may be desirable. When the suspending agent also performs the function of conditioning agent the proportion of suspending agent in the composition may be increased to compensate for the partial or total lack of a separate conditioning agent.

Cationic detergents may perform the functions of both foam-building agent and conditioning agent. When such a detergent is used as a foam-building agent in the compositions of the present invention it is not necessary to include a separate conditioning agent, although this may be desirable. When the foam-building agent also performs the function of conditioning agent, the proportion of foam-building agent in the composition may be increased to compensate for the partial or total lack of a separate conditioning agent.

The conditioner may also comprise various conventional accessory ingredients such as perfumes colouring agents, preservatives and thickeners.

- 5 -

The conditioner may be produced by methods conventional in the art and the pH of the final formulation adjusted to be in the range pH 5 to 8.5, preferably pH 6 to 7.5. Such adjustment is preferably effected by addition of citric acid or sodium hydroxide, as appropriate.

The conditioner of the present invention may be presented in any conventional form such as a suspension, gel, paste or emulsion, which may be produced by methods conventional in the art. When presented as a suspension the conditioner is formulated with the above-described components in the specified proportions. When formulated as an emulsion however, the conditioner is formulated as described above and then emulsified with a cosmetically acceptable oil, generally as an oil-in-water emulsion. In this case the aqueous phase contains the components specified above in the specified proportions. The surfactants already in the aqueous suspension may be sufficient to act as emulsifying agent; if not additional emulsifying agent may be included as necessary, up to about 10% w/w of the final emulsion weight.

The conditioner may be presented in any of the conventional containers, such as in bottles, tubes and sachets. However, it is particularly preferred that the conditioner be presented in a two part pack in association with a conventional shampoo. One part of the pack contains shampoo and the second part the conditioner of the present invention. The pack, therefore, affords a complete hair cleaning, conditioning and dandruff-controlling treatment. Generally one application uses about 10-25, usually about 12-15 $cm^3$ of shampoo or conditioner, thus each part of the pack may conveniently contain a multiple of such a volume.

According to the present invention in a particular embodiment there is, therefore, provided a two part hair treatment pack which comprises in the first part, a conventional shampoo and in the second part a medicated foaming conditioner, as hereinbefore defined.

The conventional shampoo may be medicated or non-medicated.

According to the present invention there is also provided a method for conditioning the hair and treating or preventing dandruff, which method comprises applying a medicated foaming conditioner, as hereinbefore defined, to hair which has been cleaned with a conventional shampoo.

The present invention will now be illustrated with reference to the following Examples, which are not intended to limit the invention in any way:

Example 1

Medicated Foaming conditioner

|  | % w/w |
|---|---|
| Cetyl trimethyl ammonium bromide | 10.00 |
| Hydroxyethyl cellulose | 1.00 |
| Sodium hydroxide | 0.15 |
| Quaternised hydroxyethyl cellulose | 0.50 |
| 2-bromo-2-nitropropane-1,3-diol | 0.01 |
| Zinc pyridinethione | 0.35 |
| Perfume | q.s |
| Dyes | q.s |
| Water to | 100.00 |

Example 2

Medicated Foaming conditioner

|  | % w/w |
|---|---|
| Stearyl dimethyl benzyl ) Ammonium chloride ) | 1.00 |
| Hydroxyethyl cellulose | 1.00 |
| Sodium hydroxide | 0.15 |
| Quaternised hydroxyethyl cellulose | 0.50 |
| 2-bromo-2-nitropropane-1,3-diol | 0.01 |
| Zinc pyridinethione | 0.35 |
| Sodium lauryl sarcosinate | 3.00 |
| Perfume | q.s |
| Dyes | q.s |
| Water to | 100.00 |

## Example 3

### Medicated Foaming conditioner

|  | % w/w |
| --- | --- |
| Stearyl dimethyl benzyl ammonium chloride | 1.00 |
| Hydroxyethyl cellulose | 1.00 |
| Sodium hydroxide | 0.15 |
| Quaternised hydroxyethyl cellulose | 0.50 |
| 2-bromo-2-nitropropane-1,3-diol | 0.01 |
| Zinc pyridinethione | 0.35 |
| Alkyl dimethyl polyoxy amine oxide | 5.00 |
| Perfume | q.s |
| Dyes | q.s. |
| Water                          to | 100.00 |

## Example 4

### Medicated Foaming conditioner

|  | % w/w |
| --- | --- |
| Cetyl trimethyl ammonium chloride | 1.00 |
| Hydroxyethyl cellulose | 1.00 |
| Sodium hydroxide | 0.15 |
| Quaternised hydroxyethyl cellulose | 0.50 |
| 2-bromo-2-nitropropane-1,3-diol | 0.01 |
| Zinc pyridinethione | 0.35 |
| Alkyl dimethyl betaine | 5.00 |
| Perfume | q.s |
| Dyes | q.s |
| Water                          to | 100.00 |

Example 5

A medicated foaming conditioner as described in Examples 1-4 may be packed in one part of a two-part pack. The other part may contain a conventional shampoo such as a formulation of the following composition:-

### ORDINARY SHAMPOO

|  | % w/w |
|---|---|
| Sodium lauryl ether sulphate | 17.00 |
| Coconut diethanolamide | 4.00 |
| Ethylene glycol monostearate | 2.00 |
| 2-bromo-2-nitropropane-1,3 diol | 0.01 |
| Dyes | q.s |
| Perfume | q.s |
| Sodium chloride | q.s |
| Citric acid | q.s |
| Water to | 100.00 |

### MEDICATED SHAMPOO

|  | % w/w |
|---|---|
| Sodium lauryl ether sulphate | 17.00 |
| Coconut diethanolamide | 4.00 |
| Ethylene glycol monostearate | 2.00 |
| 2-bromo-2-nitropropane-1,3 diol | 0.01 |
| Bentonite (Suspending agent) | 0.80 |
| Zinc pyridinethione | 1.00 |
| Sodium chloride | q.s |
| Citric acid | q.s |
| Dyes | q.s |
| Perfume | q.s |
| Water to | 100.00 |

EXAMPLE 6

Medicated Foaming Conditioner.

|  | % w/w |
|---|---|
| Cetyl trimethyl ammonium bromide | 1.00 |
| Hydroxyethyl cellulose | 1.00 |
| Sodium hydroxide | 0.016 |
| Quaternised hydroxyethyl cellulose | 0.50 |
| Polyoxypropylene (9) methyl diethyl ammonium chloride | 0.50 |
| 2-bromo, 2-nitropropane, 1,3-diol | 0.01 |
| Sodium lauroyl sarcosinate | 3.00 |
| Zinc pyridinethione | 0.70 |
| Anti-oxidant | 0.10 |
| U.V. absorber | 0.09 |
| Dyes | qs |
| Perfume | qs |
| Water to | 100.00 |

CLAIMS

1.  A medicated foaming conditioner composition for conditioning the hair and for clearing or preventing dandruff, which composition comprises:

    zinc pyridinethione;
    one or more cationic suspending agents;
    one or more foam building agents; and, if necessary,
    one or more cationic conditioning agents.

2.  A conditioner as claimed in claim 1 comprising from 0.01 to 5.0 w/w zinc pyridinethione.

3.  A conditioner as claimed in claim 2 comprising from 0.1 to 1.0% w/w zinc pyridinethione.

4.  A conditioner as claimed in any one of claims 1 to 3 comprising from 0.1 to 15% of foam building agent.

5.  A conditioner as claimed in claim 4 comprising from 1 to 5% w/w of foam building agent.

6.  A conditioner as claimed in any one of claims 1 to 5 comprising 0.1 to 10% w/w of cationic conditioning agent.

7.  A conditioner as claimed in claim 6 comprising 1 to 5% w/w of cationic conditioning agent.

8.  A conditioner as claimed in any one of claims 1 to 7 wherein the suspending agent is high molecular weight quaternised hydroxyethyl cellulose, the foam building agent is sodium lauroyl sarcosinate and the conditioning agent is selected from stearyl dimethyl benzyl ammonium chloride, distearyl dimethyl ammonium chloride, cetyl trimethyl ammonium chloride, cetyl ethyl morpholinium 2-etho-sulphate, mink-amido-propyl dimethyl 2-hydroxyethyl ammonium chloride and N-stearyl colamino formyl methyl pyridinium chloride and the corresponding bromides.

9.  A hair treatment pack comprising a first part containing a conventional shampoo and a second part containing a medicated foaming conditioner as claimed in any one of claims 1 to 8.

10. A process for producing a medicated foaming conditioner as claimed in any one of claims 1 to 8, which process comprises bringing into association the zinc pyridinethione, cationic suspending agent, foam building agent and, if necessary, the cationic conditioning agent.